(19) Europäisches Patentamt
European Patent Office
Office européen des brevets

(11) EP 2 062 588 A1

(12) EUROPEAN PATENT APPLICATION
published in accordance with Art. 153(4) EPC

(43) Date of publication:
27.05.2009 Bulletin 2009/22

(51) Int Cl.:
A61K 36/06 (2006.01)  A23K 1/16 (2006.01)
A23L 1/30 (2006.01)  A61P 17/00 (2006.01)
A61P 17/16 (2006.01)

(21) Application number: 06783008.3

(22) Date of filing: 24.08.2006

(86) International application number:
PCT/JP2006/316654

(87) International publication number:
WO 2008/023425 (28.02.2008 Gazette 2008/09)

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK RS

(71) Applicant: Kirin Holdings Kabushiki Kaisha
Tokyo 104-8288 (JP)

(72) Inventors:
• YOSHIDA, Michiko
Takasaki-shi, Gunma 370-1295 (JP)
• KONISHI, Yutaka
Yokohama-shi, Kanagawa 236-0004 (JP)

(74) Representative: Wiedemann, Peter et al
Hoffmann - Eitle
Patent- und Rechtsanwälte
Arabellastrasse 4
81925 München (DE)

(54) **COMPOSITION FOR AMELIORATION OF SKIN CONDITION**

(57) This invention relates to a composition for improving skin conditions comprising, as an active ingredient, a yeast cell wall fraction obtained by removing soluble intracellular component from an enzymatically treated and/or non-enzymatically treated yeast. The composition of this invention is effective for improving skin conditions, such as, moistening, whitening, or active oxygen elimination.

Fig. 5

**Description**

Technical Field

The present invention relates to a composition for improving skin conditions comprising, as an active ingredient, a yeast cell wall fraction. More particularly, the present invention relates to a composition for improving skin conditions comprising, as an active ingredient, a yeast cell wall fraction obtained by removing soluble intracellular components from an enzymatically treated and/or non-enzymatically treated yeast.

Background Art

Every person aspires to have beautiful skin, or more specifically, a healthy skin condition. Due to the factors such as aging, ultraviolet rays, and environmental changes, however, various problems occur, such as age spots, wrinkles, and skin roughness. For the purpose of overcoming such problems, a variety of techniques have been developed in the forms of, for example, cosmetic products, oral medicine, and food. Such techniques are intended to reproduce healthy skin by temporarily supplementing a component that has been decreased inside the skin cell. They could also be intended to reproduce healthy skin by temporarily covering the skin surface, for example. Use of agents for improving skin conditions could result in a temporarily problem-free healthy skin condition.

Examples of agents that can bring about skin-whitening effects via application to the skin include a skin-whitening cosmetic product comprising, as an active ingredient, proanthocyanidin (JP Patent Publication (kokai) No. H2-134309 A (1990)), and a plant extract of *Schema, Theaceae*, as a functional component that can exhibit effects such as skin whitening and the recovery of skin resilience (JP Patent Publication (kokai) No. H9-95420 A (1997)).

As examples of skin-moistening effects or dermatologic bases, JP Patent Publication (kokai) No. 2005-15348 A discloses a skin care cosmetic product comprising β-glucan derived from microorganisms. Further, JP Patent Publication (kokai) No. 2005-162663 A discloses dermatologic bases comprising β-glucan derived from microorganisms with specified molecular weights and viscosity. JP Patent Publication (kokai) No. 2005-185187 A discloses that use of a skin lotion comprising a water-soluble fraction of a yeast cell wall that achieves effects of skin moistening. JP Patent Publication (kokai) No. 2001-163720 A discloses a moistening agent, a cosmetic agent, and an external skin agent comprising mannose.

Meanwhile, food products comprising, as functional materials, ascorbic acid, *du zhong*, ginseng, Coix lacryma-jobi extract, ceramide, collagen, hyaluronic acid, chondroitin sulfate, DNA, or the like have been developed, including, for example, a metabolism accelerator comprising, as an essential component, collagen protein or hydrolysate thereof (JP Patent Publication (kokai) No. H7-278012 A (1995)) and a health food product comprising, as an active ingredient, ceramide (JP Patent Publication (kokai) No. H11-113530 A (1999)).

In recent years, active oxygen has drawn attention as a cause of skin roughness or skin aging. Active oxygen is generated during the process of energy metabolism in biological cells, and it is known as "superoxide" (i.e., superoxide anion, hydrogen peroxide, single oxygen, hydroxy radical, or the like resulting from one-electron reduction of an oxygen molecule). Such active oxygen is essential for the microbicidal mechanism of phagocytic cells, and it plays a key role in removal of viruses or cancerous cells. Excessively generated active oxygen, however, attacks biological molecules that constitute membranes or tissue within the body and then induces various diseases. For example, active oxygen is considered to degrade, denature, or crosslink biological tissue, such as collagen, or to generate lipid peroxide, which would oxidize oils and fats and damage cells. Damages induced by active oxygen are considered to cause skin aging, such as wrinkling or lowered skin resilience.

The radical firstly generated from oxygen in the body is superoxide, and other radicals such as hydroxy radicals are generated through superoxide. A superoxide in the cell is converted into hydrogen peroxide by superoxide dismutase (SOD) generated in the cells. The amount of SOD decreases along with aging, the decreased amount of SOD results in an elevated superoxide concentration in the cell, and superoxide eventually damages the body. In order to supplement the decreased amount of SOD, SOD itself, tocopherols, a scutellaria root extract, or the like is used as an SOD-like agent. Also, a composition for suppressing elevation of lipid peroxide levels comprising manno-oligosaccharide of a given degree of polymerization has been reported (JP Patent Publication (kokai) No. 2002-262828 A).

A yeast cell wall fraction obtained by removing soluble intracellular components from yeast is already known. Various types of yeast cell wall fractions, depending on methods for producing the same, are known. Depending on production methods, yeast cell wall fractions having various compositions of a dietary fiber content, protein content, and the like, are known (JP Patent Publication (kokai) No. 2001-55338 A and JP Patent Publication (kokai) No. 2002-153263 A). Also, various physiologically active functions of such yeast cell wall fractions are known. For example, various functions, such as prevention of constipation and/or amelioration of symptoms thereof, prevention of allergic diseases and/or amelioration of symptoms thereof (JP Patent Publication (kokai) No. 2001-55338 A), syneresis preventive effects (JP Patent No. 3502341), prevention and improvement of decreased renal function (JP Patent Publication (kokai) No.

2004-161618 A), acceleration of mineral absorption, and prevention and improvement of osteoporosis (JP Patent Publication (kokai) No. 2004-292382 A), are known. However, effects of improving skin conditions via oral dosing with such yeast cell wall fractions are not yet known.

Disclosure of the Invention

Problems to be Resolved by the Invention

As described above, many techniques intended for skin moistening or whitening have been developed, although effects of such techniques are often insufficient. Attempts to overcome such problems have been made by, for example, a means of increasing the amount of an active ingredient to be mixed. Such attempts, however, impose problems in terms of cost and stability in pharmaceutical products, and improvement of the intended effects has been strongly desired. For example, JP Patent Publication (kokai) No. 2005-15348 A, JP Patent Publication (kokai) No. 2005-162663 A, JP Patent Publication (kokai) No. 2005-185187 A, and JP Patent Publication (kokai) No. 2001-163720 A disclose external agents comprising a water-soluble active ingredient. Such cosmetic products to be applied to the skin cannot maintain their effects because the effective ingredient would be eliminated over time due to sweating. Thus, effects of preventing skin aging are disadvantageously insufficient, or effects are exhibited only at a site of the skin to which the agent has been applied.

In order to resolve a problem resulting from such external influence, food products intended for improving skin conditions that are to be orally ingested have been developed. In such a case, however, there are concerns about the possibility of allergic symptoms caused by an active ingredient due to oral ingestion, and unexpected side effects due to influence on metal ions, which are essential for functions of digestive enzymes, or the like. Further, potential problems due to oral ingestion, for example, insufficient effects on the skin due to degradation and modification of an active ingredient by a gastric acid or digestive enzyme, lowered digestion and absorption in the intestinal tract, or the like, have been pointed out.

That is, currently available products intended for improving skin conditions that can be used to be applied to the skin or administered orally are not sufficient in terms of functions and safety.

SOD, which is an enzyme in the body and a substance for eliminating active oxygen, is very expensive because its purification is difficult, and it is thermally unstable and easily becomes inactive. Thus, an alternative substance for SOD (i.e., an SOD-like active material), that exhibits the same active oxygen-elimination effect as SOD, is highly stable, and is cost effective, has been awaited.

The present invention is intended to discover a substance having effects of moistening and/or whitening skin (i.e., effects of suppressing pigmentation) and/or effects of eliminating active oxygen with the SOD-like activity from among naturally occurring substances with high safety. The present invention is also intended to provide a moistening composition, a whitening composition, and a composition for eliminating active oxygen utilizing such substances.

Means for Resolving the Problems

In order to overcome the above-mentioned problems, the present inventors have conducted concentrated studies regarding naturally occurring substances having effects of improving skin conditions that are safe and exhibit excellent effects of improving skin conditions. As a result, the present inventors have now found that oral feeding with a yeast cell wall fraction obtained by removing soluble intracellular components from an enzymatically treated and/or non-enzymatically treated yeast would surprisingly exhibit excellent effects of improving skin functions, and also that such fraction could be used as a safe and no side-effect composition for improving skin conditions. This has led to the completion of the present invention.

As a yeast cell wall fraction used for the composition for improving skin conditions of the present invention, a yeast cell wall fraction obtained by further subjecting the yeast cell wall fraction obtained by removing soluble intracellular components from enzymatically treated and/or non-enzymatically treated yeast to water washing, or to both high-pressure treatment and water washing, can be used as a particularly preferable yeast cell wall fraction in the present invention.

A yeast cell wall fraction that is used as an active ingredient of the composition for improving skin conditions of the present invention is prepared by removing soluble intracellular components from a yeast and purifying the resultant to bring the dietary fiber content in dry matter of the yeast cell wall fraction to preferably 60% by weight or more, more preferably 65% by weight or more, and most preferably 80% by weight or more.

The composition of the present invention for improving skin conditions comprising the yeast cell wall fraction is derived from naturally occurring products, and is safe, free of side effects, and safe for administration to the body. Administration of said composition can bring about effects of improving skin conditions. Also, the composition for improving skin conditions of the present invention can exhibit excellent effects of improving skin conditions in a wide variety of applications, such as pharmaceutical products, food or beverage products, or feeds.

Summary of the Invention

In summary, the present invention encompasses the following features:

(1) A composition for improvement of skin conditions comprising, as an active ingredient, a yeast cell wall fraction obtained by removing soluble intracellular components from an enzymatically treated and/or non-enzymatically treated yeast.

(2) The composition according to (1), wherein the active ingredient is a yeast cell wall fraction obtained by further subjecting the yeast cell wall fraction obtained by removing soluble intracellular components from an enzymatically treated and/or non-enzymatically treated yeast, to water washing, or to both high-pressure treatment and water washing.

(3) The composition according to (1) or (2), wherein the dietary fiber content of the yeast cell wall fraction is 60% by dry weight or more.

(4) The composition according to any of (1) to (3), which is fed or administered to an animal.

(5) The composition according to any of (1) to (4), wherein the improvement of skin conditions is a skin-moistening effect.

(6) The composition according to any of (1) to (4), wherein the improvement of skin conditions is a skin-whitening effect.

(7) The composition according to any of (1) to (4), wherein the improvement of skin conditions is an active oxygen-removing effect.

(8) The composition according to any of (1) to (7), which is a pharmaceutical product, food or beverage product, pet food, or feed.

(9) A method for improving skin conditions of an animal comprising feeding or administering the composition according to (1) to the animal.

(10) Use of a yeast cell wall fraction obtained by removing soluble intracellular components from an enzymatically treated and/or non-enzymatically treated yeast, for producing a composition for improving skin conditions.

Definition of Terms

The terms used herein are defined as follows.

The term "yeast cell wall fraction" refers to a fractionated substance mainly composed of a yeast cell wall obtained by removing intracellular components (e.g., proteins, amino acids, nucleic acids, saccharides, etc.) that are soluble in water or a polar solvent (e.g., an aqueous alcohol or acetone) from enzymatically-treated and/or non-enzymatically treated yeast cells. More specifically, the yeast cell wall fraction can be obtained by subjecting a yeast cell wall fraction obtained by removing soluble cell components from an enzymatically treated and/or non-enzymatically treated yeast or a yeast extract residue discharged from conventional production of a yeast extract, to water washing, or to both high-pressure treatment and water washing (see Examples 1 and 2 below). The yeast cell wall fraction is **characterized in that**, for example, the dietary fiber content is 60% by dry weight or more, preferably 65% by dry weight or more, and most preferably 80% by dry weight or more.

The term "high-pressure treatment" refers to a treatment for effectively further removing contaminants, such as soluble intracellular components, from a yeast cell wall fraction obtained by removing soluble intracellular components from an enzymatically treated and/or non-enzymatically treated yeast, including treatments with the use of a homogenizer, French press, or extruder under high pressure.

The term "dietary fiber" refers to a fibrous component that is less likely to be digested by human digestive enzymes. In the present invention, this term refers particularly to a water-insoluble dietary fiber.

The term "skin condition(s)" refers to unhealthy skin conditions of an animal (preferably a human), such as skin roughness, cracked skin, loss of firmness, or wrinkles resulting from skin dryness, pigmentation, age spots, or freckles resulting from sunburn or the like, dermatological diseases caused by active oxygen (except for atopic dermatitis), and the like.

The term "skin-moistening effect" refers to an effect of suppressing evaporation of moisture from the skin surface and retaining moisture.

The term "skin-whitening effect" refers to an effect of suppressing melanin production; i.e., pigmentation.

The term "active oxygen-elimination effect" refers to an effect of decreasing active oxygen or suppressing generation of active oxygen (also referred to as "superoxide") in the body.

The term "feeding" or "ingestion" refers to sending of a food or beverage product or a drug into the gastrointestinal tract through the mouth or esophagus. This term generally refers to oral feeding or oral ingestion, or oral administration. The term "administration" refers to oral administration.

The term "animals" refers to mammalian animals, such as humans, pets (e.g., dogs or cats), or livestock animals (e.g., bovine, horse, sheep, or goat), preferably humans.

Effects of the Invention

The composition of the present invention has effects of improving skin conditions, including skin-moistening effects, skin-whitening effects, and active oxygen elimination effects. Accordingly, such composition can be used for a pharmaceutical product, food or beverage product, or feed for improving skin conditions. The yeast cell wall fraction as an active ingredient of such composition is derived from naturally occurring products, and is safe and has no side effects. Thus, the cell wall fraction can be safely administered into the body. Further, effects of improving skin conditions which said active ingredient has are actually verified to provide significant effects in humans and rats in the examples below.

Brief Description of the Drawings

Fig. 1 shows the amount of transepidermal moisture evaporation in human subjects after the feeding of a brewer's yeast cell wall fraction (BYC).
Fig. 2 shows the melanin level in the submandibular area of human subjects after the dosing of BYC.
Fig. 3 shows the melanin level in the cheek area of human subjects after the dosing of BYC.
Fig. 4 shows changes in cheek moisture content over time in human subjects, resulting from the feeding of BYC.
Fig. 5 shows changes in the amount of cheek moisture evaporation over time in human subjects, resulting from the feeding of BYC.
Fig. 6 shows the effect of the feeding of BYC on age spots on the face of a human subject (one male).
Fig. 7 shows changes in skin moisture content for a group of rats to which BYC has been administered, compared with a control group.
Fig. 8 shows changes in the amount of transepidermal moisture evaporation for a group of rats to which BYC has been administered, compared with a control group.
Fig. 9 shows changes in melanin levels for a group of rats to which BYC has been administered, compared with a control group.
Fig. 10 shows percentage of superoxide elimination for the group of rats to which YCW has been administered and for the group of rats to which BYC has been administered.

Best Modes for Carrying out the Invention

The present invention relates to a composition for improving skin conditions comprising, as an active ingredient, a yeast cell wall fraction obtained by removing soluble intracellular components from an enzymatically treated and/or non-enzymatically treated yeast.
A particularly preferable example of the yeast cell wall fraction is a yeast cell wall fraction, which is obtained by further subjecting the yeast cell wall fraction obtained by removing soluble intracellular components from an enzymatically treated and/or non-enzymatically treated yeast to water washing, or to both high-pressure treatment and water washing. A yeast cell wall fraction that is used as an active ingredient of the composition for improving skin conditions of the present invention is prepared by removing soluble cell components from a yeast and purifying the resultant to bring a dietary fiber content of the yeast cell wall fraction to preferably 60% by dry weight or more, more preferably 65% by dry weight or more, and most preferably 80% by dry weight or more, in order to exhibit excellent effects of improving skin conditions.
Hereafter, the present invention is described in more detail.

(Starting yeast)

A yeast that serves as a starting material for the yeast cell wall fraction used in the present invention is not particularly limited, as long as it taxonomically belongs to yeast and is edible. For example, a brewers' yeast, which is a by-product of the brewing process, wine yeast, baker's yeast, torula yeast, alcohol yeast, or Japanese "Sake" yeast can be used. Specific examples include, but are not limited to, *Saccharomyces cerevisiae, Saccharomyce bayanus, Saccharomyces pastorianus*, and *Saccharomyces carlsbergensis* of *Saccharomyces* to which brewer's yeast and baker's yeast belong, and other yeast cells, such as *Zygosaccharomyces rouxii, Candida utilis*, and *Candida lipolytica*.
In the present invention, the above-mentioned yeasts can be used alone or in combination. Use of live yeast is preferable. When other forms of yeast other than live yeast, such as dry yeast, are used, for example, such yeast may be suspended in water or the like and treated in the same manner as is the case of live yeast. Further, the form or size of yeast to be used is not particularly limited. The size is preferably between 1 μm and 20 μm.

(Preparation of yeast cell wall fraction)

In the present invention, the yeast cell wall fraction used as an active ingredient is obtained by removing intracellular components that are soluble in water or a polar solvent, such as proteins, amino acids, nucleic acids, and saccharides, from enzymatically treated and/or non-enzymatically (i.e., without use of an enzyme) treated yeast cells. In general, such fraction can be obtained by lysing yeast cells via enzyme treatment, and separating and removing soluble intracellular components from the cells.

Examples of such enzyme treatment technique include a so-called autodigestion involving the use of an enzyme from yeast, addition of an enzyme, such as protease, nuclease, glucanase, or esterase, from the outside, and a combination thereof. The yeast cell wall fraction can be obtained by removing soluble intracellular components from said enzymatically-treated yeast cells via centrifugation or other means. Preferably, the resulting yeast cell wall fraction may further be subjected to both high-pressure treatment and water washing to obtain a yeast cell wall fraction with higher purity.

Examples of the non-enzymatic-treatment include mechanical procedures, such as mechanical grinding, osmotic pressure difference, freezing-thawing, ultrasonication, and pressurization. Mechanical grinding is a technique involving mechanical grinding of yeast in an aqueous solution, such as water or buffer, using a grinder, such as a blender, ball mill, or homogenizer. The osmotic pressure difference is a technique utilizing differences in osmotic pressure to destroy the cell wall. For example, yeast cells are suspended in a hypotonic solution in order to destroy the cells. Freezing-thawing is a technique involving repeating the cycle of freezing and thawing of yeast in order to destroy the cell wall: Ultrasonication is a technique involving placing yeast cells into, for example, ultrasounds of 10 to 600 kc/sec in order to destroy the cells. The pressurization technique is a technique involving destroying cells via rapid pressure changes, for example, by instantaneous application of a pressure of, for example, 900 psi or higher with the use of a press in a sealed container containing yeast cells, or by ejecting cells pressurized at approximately 20,000 psi from a small nozzle.

Since all of the above treatment techniques are used in the production of a yeast extract (soluble intracellular components), a yeast extract residue, which is a by-product in the yeast extract production, is advantageously used as a yeast cell wall fraction in view of production costs.

The yeast extract residue can be obtained by, for example, a method for producing a yeast extract, comprising degrading an aqueous suspension of heat-inactivated yeast with an enzyme, removing a water-insoluble matter with the addition of chitosan or both of chitosan and polyacrylate, and then obtaining an aqueous solution of a yeast extract, as disclosed in JP Patent Publication (kokai) No. H8-56611 A (1996), a method for producing a yeast extract, comprising adjusting a pH of a suspension of yeast cells that have been treated with a high-pressure homogenizer to a neutral from weak alkaline level (e.g., a pH level of about 9 or lower) and adding an enzyme agent containing endo-type protease to the suspension of yeast cells for autodigestion, as disclosed in JP Patent Publication (kokai) No. H9-56361 A (1997), or a method for producing a yeast extract, comprising heating yeast cells (as a raw material), and digesting the cell components under conditions where nuclease is present and enzyme activity for degrading 5'-nucleotide is suppressed followed by extraction to obtain a yeast extract, as disclosed in JP Patent Publication (kokai) No. H11-332511 A (1999). Also, a yeast extract residue resulting from other known techniques for producing a yeast extract can be effectively used.

Use of a yeast cell wall fraction resulting from water washing or both high-pressure treatment and water washing is preferable because of superior effects of improving skin conditions. Further, use of such fraction is preferable since such fraction would not develop any unfavorable distinctive taste or odor due to autodigestion and thus is suitable for oral ingestion.

High-pressure treatment can be carried out by any method, provided that soluble intracellular components can be effectively removed. Examples of such methods include a treatment with a high-pressure homogenizer, a treatment with French press, and a treatment with an extruder. Among high-pressure treatment techniques, the treatment with a high-pressure homogenizer is the most preferable from the viewpoint of efficient removal of contaminants. In the case of using a high-pressure homogenizer, high-pressure treatment of a yeast cell wall fraction obtained by removing soluble intracellular components from an enzymatically treated and/or non-enzymatically treated yeast includes a high-pressure treatment under the pressure of, preferably 100 to 1,500 kg/cm$^2$, and more preferably at 500 to 1,500 kg/cm$^2$. In order to further enhance the effect of removal of contaminants, the yeast cell wall fraction obtained by removing soluble intracellular components from enzymatically treated and/or non-enzymatically treated yeast may be subjected to plural high-pressure treatments, or a combination of two or three different types of high-pressure treatment techniques as described above may optionally be performed.

Water washing following high-pressure treatment of a yeast cell wall fraction obtained by removing soluble intracellular components from enzymatically treated and/or non-enzymatically treated yeast may be carried out by any technique, provided that soluble intracellular components can be separated from a water-insoluble yeast cell wall fraction. Examples of such techniques include centrifugation and filtration, and centrifugation is more preferable from the viewpoint of operating efficiency. Water which can be used in water washing includes, in addition to general clean tap water, acidic water, alkaline water, or water containing an alcohol (e.g., ethanol) may be used, provided that the efficiency to remove contaminants is not reduced. The number of times of washing may be appropriately determined depending on the extent or degree of removal of contaminants.

Following the high-pressure treatment and water washing, a treatment with an organic solvent, a treatment with an enzyme, a treatment with an acid or alkali, or the like may be performed to produce a more purified yeast cell wall fraction, if necessary.

The yeast cell wall fraction obtained by the above methods can further be subjected to drying. Drying can be, for example, freeze drying, spray drying, air drying, hot air drying, or the like.

The yeast cell wall fraction of the present invention is prepared to comprise dietary fiber of generally 60% by weight or more, preferably 65% by weight or more, and further preferably 80% by weight or more, in dry matter.

(Measurement of dietary fiber content)

The dietary fiber content is determined by the enzymatic-gravimetric method as described bellow.

Specifically, 1 g of the sample is aliquoted to each of two 500-ml tall beakers such that a difference in the amount of the samples falls within 20 mg (S), then 50 ml each of 0.08 mol/l phosphate buffer (pH 8.0) and 0.1 ml each of Termamyl (120 1, Novo Nordisk) are added, and the resulting mixtures are heated for 30 minutes with shaking at intervals of about 5 minutes in boiled water. After the mixtures are cooled, the pH is adjusted to $7.5 \pm 0.1$ with 10 ml of 0.275 mol/l sodium hydroxide, 0.1 ml each of a protease solution dissolved in phosphate buffer at 50 mg/ml (P-5380, Sigma) is added, and the resultants are shaked at 60°C for 30 minutes. After the mixtures are cooled, the pH is adjusted to $4.3 \pm 0.3$ with 10 ml of 0.325 mol/l hydrochloric acid, 0.1 ml each of an amyloglucosidase solution (A-9913, Sigma) is added, and the resultants are shaked at 60°C for 30 minutes. Thereafter, 4 volumes of 95% ethanol heated at 60°C are each added, the resultants are allowed to stand at room temperature for 1 hour, a 2G2 glass filter comprising a filter layer of about 1.1 g of celite previously formed thereon is used to perform suction filtration. The residue is washed three times with 20 ml of 78% ethanol, twice or more with 10 ml of 95% ethanol and then twice or more with 10 ml of acetone, followed by drying at 105°C overnight. After the constant weights (R1 and R2) are measured, one of the samples is subjected to ashing at 525°C for 5 hours, and an ash content (%) (A) is calculated. In the other samples, the protein content (%) (p) is determined and calculated by the Kjeldahl method (coefficient: 6.25). The values obtained in the same manner without addition of a sample are determined as blank values. Dietary fiber contents are determined based on the obtained values by the following formulae.

$$\text{Dietary fiber (\% by weight)} = (R \times (1 - (P + A)/100 - B)/S \times 100$$

R: Weight of the residue (average, (R1 + R2)/2, mg)
P: Protein content in the residue (%)
A: Ash content in the residue (%)
S: Amount of the sample collected (average, mg)
B: Blank (mg)

$$B \text{ (mg)} = r \times (1 - (p + a)/100)$$

r: Weight of the blank residue (average, mg)
p: Protein content in the blank residue (%)
a: Ash content in the blank residue (%)

A protein content is measured by the Kjeldahl method, and a protein content can be measured in accordance with, for example, the method described in the document "Shokuhin bunseki hou (Method for food analysis), edited by the committee for editing the method for food analysis, the Japanese Society for Food Science and Technology, Japan, p. 101."

(Application of compositions for improving skin condition)

The composition of the present invention has an effect of suppressing the amount of moisture evaporation from the surface of the skin (or flesh). Thus, such composition has an effect of moistening the flesh of an animal, preferably a human, and the composition also has an effect of retaining moisture. Accordingly, feeding, ingestion or administration of such composition can improve dry conditions of the skin (i.e., improvement of dry skin), prevent skin dryness (i.e., prevention from dry skin), or enhance and improve the barrier functions of the cornified layer of the skin. Such excellent effects can also be expected to impart firmness or glossiness to the skin, to improve wrinkling or sagging of the skin, and to prevent or improve the skin roughness resulting from dryness. The composition of the present invention has the skin-moistening effects. Thus, such composition is expected to exhibit effects of, for example, hydrating the skin, retaining the skin moisture, softening the skin, smoothing the skin, or polishing the skin.

Accordingly, the composition of the present invention can be preferably used for, for example, a person whose skin is dry, a person whose skin tends to become dry, a person whose skin easily becomes dry, a person whose skin is sensitive, a person who feels uneasy about cracked skin, or a person whose scalp skin is dry, for imparting the skin-moistening effects or effects of improving the barrier functions of the cornified layer of the skin.

Furthermore, feeding, ingestion or administration of the composition of the present invention to a human can prevent or alleviate various symptoms or diseases caused by (or associated with) skin dryness, such as senile xerosis or dry skin in children.

The composition of the present invention also has an effect of suppressing melanin production in humans. Accordingly, the ingestion of such composition can prevent or ameliorate pigmentation after sunburn, age spots, freckles, or liver spots, and can exhibit excellent skin-whitening effects.

Furthermore, since the composition of the present invention has an active oxygen-elimination effect in an animal, preferably a human, it can be used for, for example, prevention or treatment of dermatologic diseases caused by active oxygen ($O_2^-$), such as age spots, freckles, skin roughness, intolerance to cold, or wrinkling. The composition can also be employed in the fields of medical products, food or beverage products such as health food products, or food additives for the purpose of treatment, amelioration, prevention, or other purposes for various diseases caused by active oxygen ($O_2^-$), such as diseases resulting from blood circulation disorders (e.g., myocardial infarct, stroke, elevated blood pressure, menstrual cramps, shoulder stiffness, neuralgia, low back pain, or hangover) and lifestyle-related diseases and internal diseases (e.g., cancer, hepatitis, or diabetes).

The composition of the present invention can also be used for a feed for livestock animal or a pet food for dog, cat or the like.

Such a feed or pet food may be in any form as is the case of the above-mentioned compositions, provided that the feed or pet food is in conventional forms of feeds or pet foods, and can be combined with additives or food materials that are generally contained in feeds or pet foods to prepare various forms of feeds or pet foods in accordance with conventional techniques. Of course, the composition of the present invention may be used as such, as a feed or pet food. Also, the content of the composition of the present invention in the feed or pet food, the amount of the feed or pet food to be fed, or other conditions are not particularly limited, provided that the effects of the present invention can be exhibited. Such conditions are adequately determined in accordance with the form of the feed or pet food, types of livestock animals or pets, or other factors. For example, the conditions similar to those employed for the above-mentioned food products can be employed. When the composition of the present invention is used in a feed for livestock animal or a pet food, such a feed or pet food can be fed with the hope of achieving the aforementioned effects of improving the skin conditions, such as effects of suppressing the amount of moisture evaporation from the skin (or flesh) or effects of improving dry skin conditions accompanied therewith.

The composition of the present invention can comprise, as an active ingredient, a yeast cell wall fraction alone, or the composition can be used in the form of a mixture of a yeast cell wall fraction with other ingredients or materials for improving skin conditions. Examples of other ingredients or materials for improving skin conditions include collagen, hyaluronic acid, chondroitin sulfate, proanthocyanidin, tocophenol, and ascorbic acid.

The composition of the present invention can be formulated in the form of a pharmaceutical product, food or beverage product including a health food product, or feed, depending on the intended application. Alternatively, the formulated product can be incorporated in or mixed with food products, feeds, or the like. The composition of the present invention can be used as an agent for preventing or improving dry skin conditions, a food product for skin moistening, an agent for improving the barrier functions of the cornified layer of the skin, an antioxidant, an anti-aging agent, a food or beverage product for cosmetic purposes, a skin care cosmetic product, or a food product for skin-whitening in animals, for example.

The form of formulation may be in any form of solid (e.g., a solid, powdery, granular, or other form), paste, gel, liquid, or turbid (or suspension). When producing such formulation, an adjuvant that is commonly used for producing a solid formulation, such as a carrier, excipient, diluent, lubricant, preservative, disintegrator, suspending agent, coloring agent, flavoring agent, or sweetener, can be adequately used.

Examples of formulation include various formulations for internal use (including formulations for animals, such as livestock animals or pets). Specific examples include 1) pharmaceutical products and 2) quasi drugs, which are in the form of ampoule, capsule, pill, tablet, powder, granule, solid, liquid, gel, or air bubble.

Examples of carrier and excipient include sterilized water, lactose, magnesium stearate, starch, talc, gelatin, agar, pectin, gum Arabic, vegetable oil (e.g., olive oil and sesame oil), cacao butter, ethylene glycol, and alcohol (e.g., ethanol).

Examples of diluent include lactose, mannitol, glucose, hydroxypropylcellulose, microcrystalline cellulose, starch, polyvinylpyrrolidone, magnesium aluminometasilicate, sterilized water, alcohol (e.g., ethanol), and glycerol.

Examples of lubricant include magnesium stearate.

Example of disintegrator include calcium cellulose glycolate.

Examples of suspending agent include: glycols, such as propylene glycol and polyethylene glycol; vegetable oils, such as olive oil; alcohols, such as ethanol; and Polysorbate 80 (trademark).

Examples of preservative include benzalkonium chloride, benzethonium chloride, methylparaben, ethylparaben, and sodium sorbate.

Examples of flavoring agent include peppermint and citric acid.

Examples of sweetener include sucrose and artificial sweeteners, such as aspartame (trademark).

Tablets and pills may be coated with sugar, such as sucrose, gelatin, or hydroxypropylmethylcellulose phthalate, or with a film that dissolves in the stomach or intestine, or with two or more layers, if necessary.

In a capsule formulation, soft or hard gelatin can be used as coating agent.

It is preferable that an administration route is oral route.

When a yeast cell wall fraction, which is an active ingredient of the composition for improving skin conditions of the present invention, is to be added to or incorporated into a food or beverage product, such a food or beverage product can be in various forms. Examples include: various beverage products, such as yoghurt drink, fruit juice, vegetable juice, fruit and vegetable juice blends, milk, soy milk, alcoholic beverages, coffee, black tea, green tea, roasted tea, refined green tea, oolong tea, turmeric tea, Pu-erh tea, jasmine tea, sports drink, mineral water, bean paste (miso) soup, and soup; dairy products, such as cheese, butter, yoghurt, and yoghurt jelly; Western-style confectionaries, such as pudding, biscuit, cookie, cake, doughnut, and jelly; baked confectionaries, such as rice cracker; Japanese-style confectionaries, such as sweet jellied adzuki-bean paste, soft round rice cake stuffed with bean jam, a rice cake covered with bean jam, and kasutera (Japanese sponge cake); confectioneries, such as frozen dessert; an oral care composition, such as gum and candy; breads, such as sandwich loaf, French bread, and bread roll; noodles, such as Japanese wheat noodle and buckwheat noodle; processed fish pastes, such as boiled fish paste, tube-shaped fish paste cake, ham, and fish sausage; meat products, such as ham, sausage, and hamburger; seasoning agents, such as soybean paste, soy sauce, sauce, dressing, mayonnaise, honey, and sweetener; spices and condiments, such as curry powder, mustard powder, and ground pepper; food cooked on a griddle, such as octopus dumplings, *okonomiyaki* (Japanese-style pancake), and pan-fried noodles; soybean curd, konjac food, jelly, marmalade, chocolate spread, pickles, fish boiled in soy sauce, *furikake* (rice seasoning powder), *gyoza* (dumpling), pizza, sandwiches, hamburger, croquette, salad, and other various delicatessen products.

In addition, the yeast cell wall fraction can be used for general food or beverage products, such as food products for cosmetic purposes and health food products (e.g., for improving skin roughness or skin type).

The composition of the present invention preferably comprises an additive and a food material that are contained in general food products, depending on its form. Additives can optionally be selected, depending on its form, for example, from a sweetener, coloring agent, antioxidant, vitamins, aroma chemicals and the like.

The composition of the present invention can also be used as a food material for preparing an arbitrary food product. When the composition of the present invention is used as a food material for a food product, it may be added to a previously prepared food product, such as a commercially available beverage product.

A content of the yeast cell wall fraction in the composition of the present invention is 0.1 to 100% by weight, and preferably 1 to 30% by weight, although the content is not limited to such range. The dosage or the amount of feeding is adequately selected in accordance with a form of the food product, and the age, the sex and other conditions of a person to which the composition of the present invention would be fed. In general, the amount of the yeast cell wall fraction as an active ingredient of the composition of the present invention to be fed or administered is about 1 to 1, 000 mg, preferably about 10 to 200 mg, per kg of a body weight per day on a dry weight basis. The yeast cell wall fraction can be administered or fed once or several separate times per day.

In addition to the above composition, the present invention also provides a method for improving skin conditions of an animal, comprising feeding or administration of the compound to the animal.

The present invention also provides use of a yeast cell wall fraction obtained by removing soluble intracellular components from an enzymatically treated and/or non-enzymatically treated yeast, as an active ingredient of the composition of the present invention, for production of a composition for improving skin conditions.

Hereafter, the present invention is described in greater detail with reference to the examples, although the technical scope of the present invention is not limited thereto. Moreover, unless otherwise indicated, the weight of the yeast cells shown in the examples is of a dry weight in the actual state.

Examples

[Example 1]

[Preparation of yeast cell wall fraction 1: YCW (yeast cell wall)]

The weight of the fermented brewer's yeast slurry that was obtained as a by-product of the brewing process was accurately measured and water was added to adjust a solid content to 10% by weight therein. Sodium hydroxide was added until the pH level reached 9, centrifugation was carried out, water was added to the precipitated fraction for washing, and centrifugation was then carried out again. The suspension in which a solid content had been adjusted to 10% by weight with addition of water was subjected to treatment with a high-pressure homogenizer while cooling, and the resultant was subjected to autodigestion at 50°C for 3 hours or longer. Then, an enzyme reaction was further carried out by adding protease at 50°C overnight, and centrifugation was carried out to remove soluble intracellular components. Water was added to adjust a solid content of the resulting yeast cell wall fraction to 10% by weight, and the resultant was washed, followed by centrifugation. This procedure was repeated twice, and the resulting precipitated fraction was lyophilized, the resultant was designated as "a yeast cell wall fraction 1: lyophilized YCW," and the components thereof were analyzed. A moisture content was measured by the drying method by heating at normal pressure, which involves heating of a sample at 105°C for 5 hours under ordinary pressure (see "*Shokuhin bunseki hou (Method for food analysis)*, edited by the committee for editing the method for food analysis, the Japanese Society for Food Science and Technology, Japan, p. 4"). A protein content was measured by the aforementioned Kjeldahl method, an ash content was measured by the aforementioned direct ashing method, and a dietary fiber content was measured by the aforementioned enzymatic-gravimetric method. Measurements were performed three times, and the average of the measured values was indicated by % by weight. The analyzed components are shown in Table 1 (Example 2 should be referred to regarding BYC1 and BYC2 in the table).

Table 1

| | Lyophilized YCW | BYC1 | BYC2 |
|---|---|---|---|
| Protein | 18.2 | 8.5 | 9.5 |
| Lipid | 2.9 | 0.8 | 1.3 |
| Ash | 3.3 | 1.4 | 3.0 |
| Sugar | 9.5 | 3.0 | 0.3 |
| Dietary fiber | 66.1 | 86.3 | 85.9 |

[Example 2]

[Preparation of yeast cell wall fraction 2: BYE (brewer's yeast cell)]

The weight of the fermented brewer's yeast slurry that was obtained as a by-product of the brewing process was accurately measured and water was added to adjust a solid content to 10% by weight therein. Sodium hydroxide was added until the pH level reached 9, centrifugation was carried out, water was added to the precipitated fraction for washing, and centrifugation was then carried out again. The suspension in which a solid content had been adjusted to 10% by weight with the addition of water was subjected to treatment with a high-pressure homogenizer while cooling, the resultant was subjected to autodigestion at 50°C for 3 hours or longer. Then, an enzyme reaction was further carried out by adding protease at 50°C overnight, and centrifugation was carried out to remove soluble intracellular components. The obtained yeast cell wall fraction was subjected to high-pressure treatment using a high-pressure homogenizer (Blue-top 40.80H, APV, England) at 60°C and 900 bar. High-pressure treatment was carried out four times, the liquid products obtained by the high-pressure treatment were each diluted 4-fold with water and stirred, and then subjected to solid-liquid separation with a centrifuge (FEUX 512, Alfa Laval, Sweden), and soluble intracellular components were washed and removed. This process of washing and removal was repeated three more times to prepare a yeast cell wall fraction slurry (hereafter abbreviated to "BYE slurry"). The product obtained by subjecting BYC slurry that had been subjected to high-pressure treatment four times to lyophilization was designated as "a yeast cell wall fraction: BYC1." The product obtained by subjecting BYC slurry to spray drying was designated as "a yeast cell wall fraction: BYC2." The components in these products were analyzed in the same manner as in Example 1. The measured values are shown in Table 1.

[Example 3]

[Test of effects of improving skin conditions of human]

Six healthy females aged 25 to 35 years were employed as subjects, and 6 g of BYC2 obtained in Example 2 was fed per day. Daily feeding was continued for 3 weeks, and changes in skin conditions (moisture contents in the cornified layer, barrier functions, and melanin level) before and after feeding were evaluated using a skin analyzer. The subjects quietly waited remaining seated in the chairs for 30 minutes after washing of measurement sites, and measurement was carried out in an environmental test laboratory in which a room temperature was adjusted to 20°C to 23°C and humidity was adjusted to 40% to 50%.

1) Moisture content in cornified layer

A moisture content in the cornified layer was measured using Corneometer CM825 (Courage+Khazaka Electronic Gmbh, Germany) by the Low-Frequency Capacitance method. Sites of measurements were the forearm, the cheek, and the area around the corner of the mouth showing a visible sign of dryness. The results are shown in Table 2. The effects resulting from feeding of BYC were not observed in the cheek area where a sufficient moisture content was observed before BYC had been fed. In the forearm and the area around the corner of the mouth, however, a moisture content apparently increased after BYC had been fed. Particularly, in the area around the corner of the mouth exhibiting the sign of dryness, a moisture content significantly increased compared with a moisture content before BYC had been fed ($^*p < 0.05$).

Table 2

| | Before feeding | After feeding of BYC2 |
|---|---|---|
| Forearm | 51.7 ± 2.9 | 56.0 ± 4.2 |
| Cheek | 64.9 ± 2.4 | 60.5 ± 4.7 |
| Around the corner of mouth | 30.5 ± 4.5 | 37.8 ± 5.1* |

2) Barrier functions

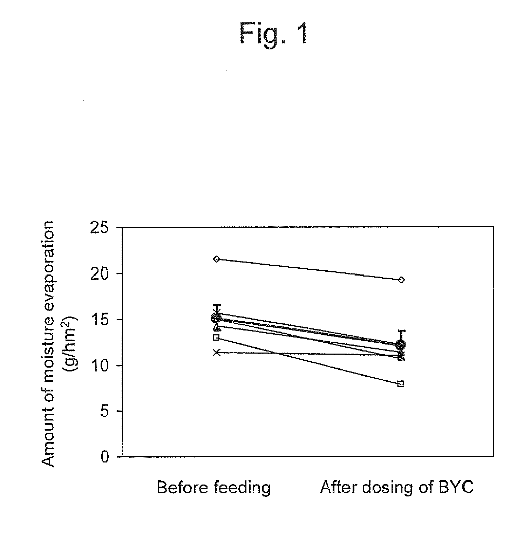

The amount of transepidermal moisture evaporation in the upper arm was measured using Tewameter TM300 (Courage+Khazaka Electronic Gmbh) and barrier functions were evaluated. The amount of transepidermal moisture evaporation is determined by measuring the amount of moisture evaporated from the skin. This indicates that a smaller value represents a higher barrier function. The results are shown in Fig. 1. As a result of feeding of BYC, lowering in the amount of transepidermal moisture evaporation was observed in substantially all subjects. While the average amount of transepidermal moisture evaporation before feeding of BYC was 14.4 g/m$^2$/hr, the value was significantly lowered to 12.1 g/m$^2$/hr after feeding of BYC ($p < 0.001$), and significant improvement was observed in barrier functions. The measured amount of transepidermal moisture evaporation is considered to represent the fact that a substance easily penetrates into the skin from the outside, as well as the fact that moisture is easily transmitted from the body to the outside. This suggests that feeding of BYC can inhibit the invasion of a stimulus from the outside.

3) Melanin level

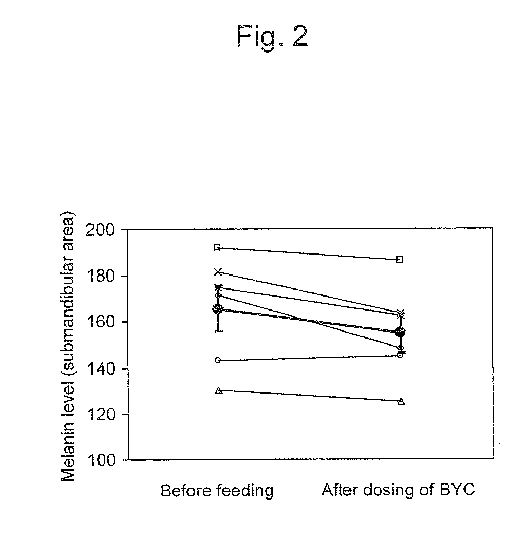

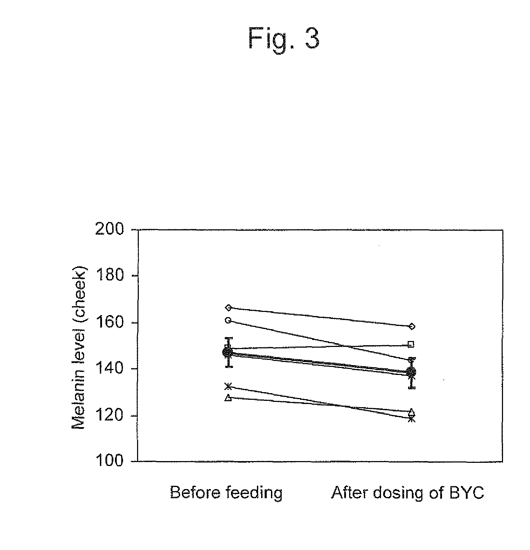

The melanin level was measured using Maxameter MX18 (Courage+Khazaka Electronic Gmbh). The sites of measurement were the submandibular area less susceptible to ultraviolet rays and the cheek susceptible to ultraviolet rays. The results are shown in Fig. 2 and Fig. 3, respectively. As a result of feeding of BYC, melanin levels were significantly lowered both in the submandibular area and in the cheek area ($p < 0.05$).

[Example 4]

[Test 2 for effects of improving skin conditions of human]

Nine males and females aged 28 to 40 years (4 males and 5 females) suffering from dry skin were employed as subjects, and 2 g of BYC2 obtained in Example 2 was fed per day. Daily feeding was continued for 3 weeks, and changes in functions of water retention in skin (a moisture content of the cornified layer and barrier function) over time were evaluated using a skin analyzer. Among the subjects, a male subject having dark age spots on the face was subjected to measurement of the melanin level to evaluate effects of feeding of BYC on dark spots. Conditions and methods of measurement were in accordance with Example 3.

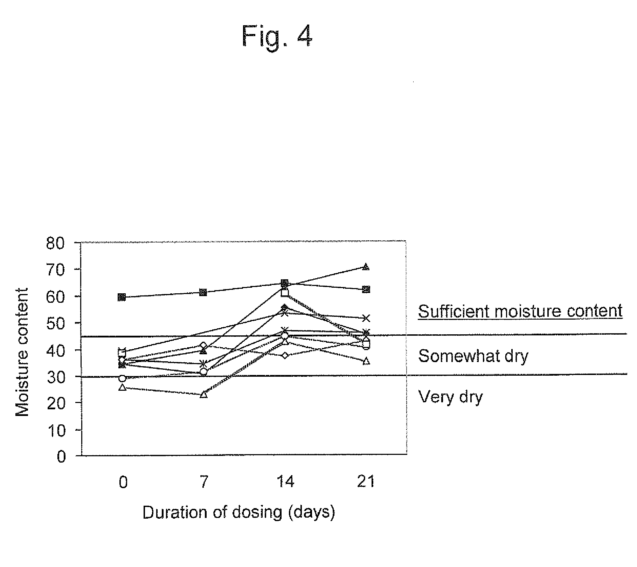

Fig. 4 shows changes in moisture contents in the cheek area of the subject with the elapse of time. Dashed lines represent male subjects and solid lines represent female subjects. As a result of feeding of BYC, increased moisture contents were observed in the cheek areas of all subjects except for a subject who retained a sufficient amount of moisture before feeding. Four female subjects who had been evaluated as "having some dryness" before feeding were evaluated as "retaining a sufficient amount of moisture" after BYC had been fed for 3 weeks. The conditions of two male subjects who had suffered from considerable dryness were improved to mild dryness.

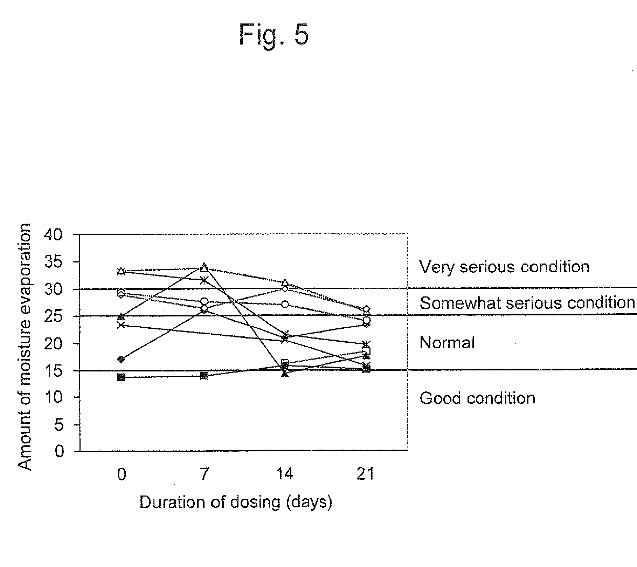

Fig. 5 shows changes in the amount of moisture evaporated from the cheek exhibiting barrier functions over time. As a result, improved barrier functions were observed in 7 of the 9 subjects, and the conditions of substantially all subjects were improved to normal levels.

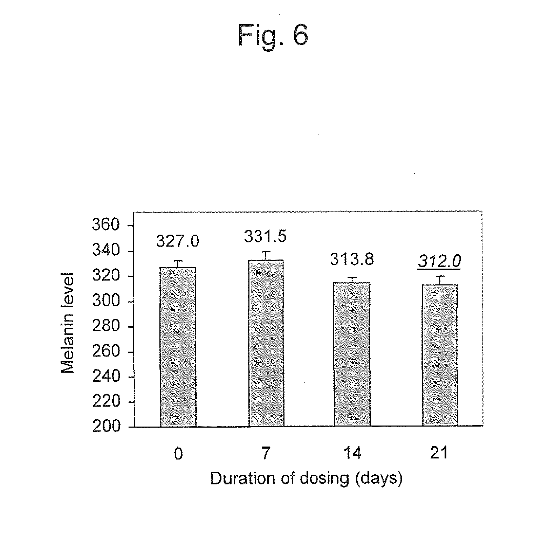

Fig. 6 shows the effects on dark spots on the face of a male subject. As a result of feeding of BYC, the melanin level at the dark spots decreased over time, and the skin whitening effects on existing dark spots were observed.

[Example 5]

[Test 3 for effects of improving skin conditions of human]

Two groups of 10 subjects (males and females in their 20's to 50's) were fed with YCW prepared in Example 1 and BYC2 prepared in Example 2 of the present invention, respectively, in amounts of 2 g per day for 2 months. Changes in their skin conditions before and after feeding were evaluated. Evaluation items were skin moistness, skin firmness (resilience), and fine wrinkles, and evaluation was made on a four-grade evaluation level: improved, somewhat improved, no change, and deteriorated; via a questionnaire. The results are shown by the number of subjects at each level in Table 3. A tendency of improvement in each parameter was observed both in the YCW group and in the BYC group. More remarkable effects were observed in the BYC group. Regarding skin moistness, a tendency of improvement was observed in substantially all subjects of the BYC group. There were no signs of side effects both in the YCW group and in the BYC group, and no deterioration was observed in skin conditions.

Table 3

| Sample | Skin moistness | | | |
|---|---|---|---|---|
| | Improved | Somewhat improved | No change | Deteriorated |
| Example 1 (YCW) | 4 | 5 | 1 | 0 |
| Example 2 (BYC2) | 6 | 3 | 1 | 0 |
| Sample | Skin firmness | | | |
| | Improved | Somewhat improved | No change | Deteriorated |
| Example 1 (YCW) | 3 | 5 | 2 | 0 |
| Example 2 (BYC2) | 5 | 4 | 1 | 0 |
| Sample | Fine wrinkles | | | |
| | Improved | Somewhat improved | No change | Deteriorated |
| Example 1 (YCW) | 2 | 5 | 3 | 0 |
| Example 2 (BYC2) | 4 | 4 | 2 | 0 |

[Example 6]

[Test for effects of improving skin conditions of hairless rat]

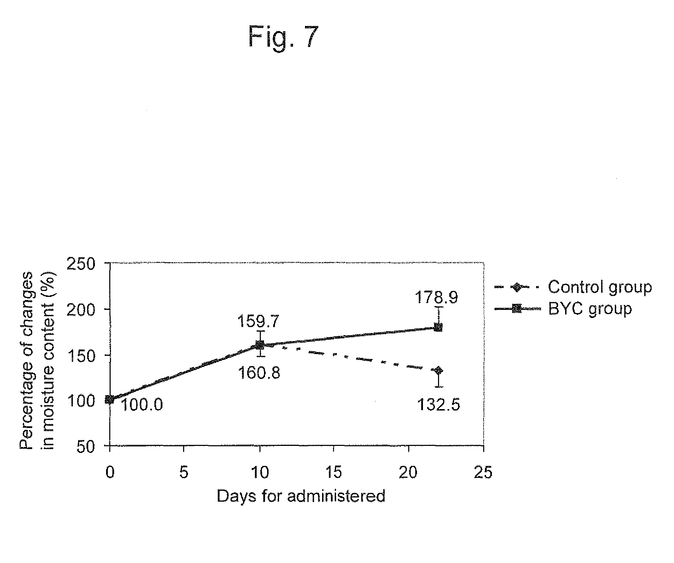

HYW/Slc male rats (5-weeks-old) were preliminarily grown under free-feeding conditions of standard powdery foods (CE-2, CLEA Japan, Inc.) for 1 week to be acclimatized to experimentation environment. Thereafter, rats were divided into two groups each consisting of 10 rats based on their body weights. The group of rats to which standard powdery foods were administered was designated as a control group. Test feeds (test samples) prepared by mixing the BYC1 samples prepared in Example 2 with the standard powdery foods at the ratio of 5%: 95% were employed. The rat subjects were allowed to freely eat these test feeds for 3 weeks, and the skin conditions (moisture contents of the cornified layer, barrier functions, and melanin level) before administration of the test feeds, on Day 10 after the initiation of administration and on Day 22 after the initiation of administration, were evaluated using a skin analyzer. The dorsal region was designated as the site of measurement and the analyzer used in Example 3 was used. The results are indicated by percentages of changes relative to the value "100" before the administration of the test feeds (Figs. 7 to 9).

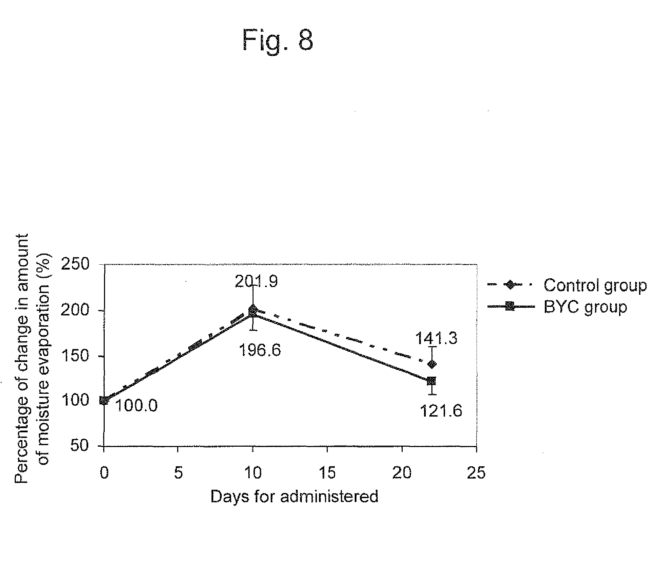

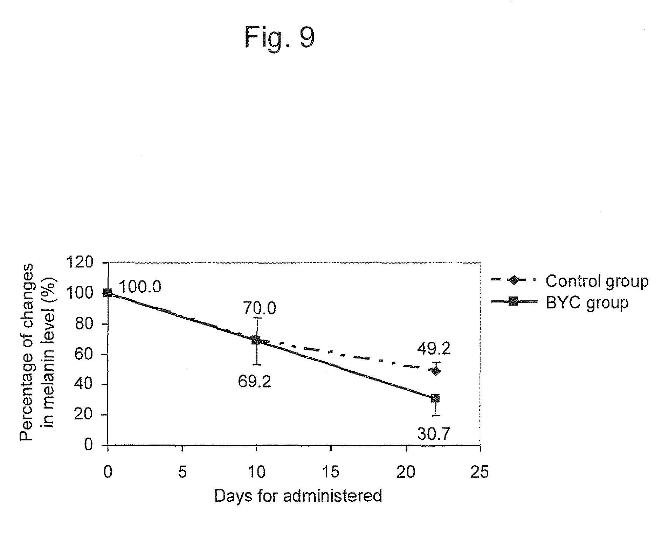

There were substantially no differences in the moisture content, the amount of transepidermal moisture evaporation, and the melanin level between the control group and the BYC group until Day 10 after the initiation of administration. The moisture content of the skin, however, increased in the BYC group compared with the control group, on Day 22 after the initiation of administration (Fig. 7). A tendency of the lowered amount of transepidermal moisture evaporation was developed in the BYC group, and enhanced barrier functions were observed as a result of feeding of BYC (Fig. 8). Further, the melanin level was notably decreased in the BYC group (Fig. 9). Thus, improved skin functions were observed in hairless rats as a result of administration of BYC feed mixtures.

[Example 7]

[Test for effect of yeast cell wall fraction on elimination of superoxide in hairless rat]

HYW/Slc male rats (5-weeks-old) were preliminarily grown under free-feeding conditions of standard powdery foods (CE-2, CLEA Japan, Inc.) for 1 week to be acclimatized to experimentation environment. Thereafter, rats were divided into three groups each consisting of 5 rats based on their body weights. The group of rats to which standard powdery foods were administered was designated as a control group. Test feeds (test samples) prepared by mixing the YCW sample or the BYC1 sample prepared in Example 1 or 2, respectively, with the standard powdery food at the ratio of 5%: 95%. The rat subjects were allowed to freely eat these test feeds for 2 weeks, the whole blood was taken from the abdominal aorta, and the blood that had been anticoagulated with ethylene-diamine-tetraacetic acid dipotassium dihydrate and the blood serum obtained via centrifugation were subjected to analysis. The SOD-like activity of the serum was assayed according to the SOD test Wako (Wako Pure Chemical Industries, Ltd.), which is based on the Nitroblue-tetrazolium (NBT) reduction test, on a 96-well microplate.

Specifically, 10 $\mu$l of the analyte was added to 100 $\mu$l of a coloring reagent containing 0.24 mM NBT and 0.4 mM xanthine, 100 $\mu$l of an enzyme solution (xanthine oxidase) was further added, and the resultant was incubated at 37°C for 20 minutes. Thereafter, the enzyme reaction was terminated with 200 $\mu$l of the reaction terminator (69 mM sodium dodecyl sulfate) and OD was measured at 560 nm using a microplate reader. The sample to which 0.1 M phosphate buffer (pH 8.0) was added instead of an enzyme was designated as a blank sample, the sample to which distilled water was added instead of the analyte was designated as a control sample, and the percentage of superoxide anion ($O_2^-$) elimination was determined by the following formulae.

$$\text{Percentage of } O_2^- \text{ elimination (\%)} = (A - B)/A \times 100$$

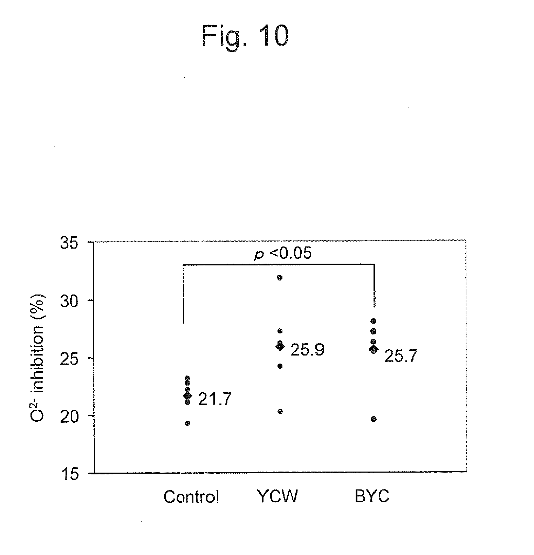

A: absorbance of the control
B: absorbance when the sample was added
The results are shown in Fig. 10.

Compared with the control sample, the percentages of superoxide elimination increased in the YCW group and in the BYC group. Particularly strong SOD-like activity was observed in the BYC group ($p < 0.05$).

[Example 8]

[Production of food or beverage product containing yeast cell wall fraction]
(1) Yoghurt jelly containing the yeast cell wall as shown in Table 4 was produced using [BYC1] prepared in Example 2.

Table 4

| Yoghurt jelly containing yeast cell wall | |
|---|---|
| Plain yoghurt | 200 ml |
| Milk | 50 ml |
| Sucrose | 60 g |
| Powdered gelatin | 15 g |

(continued)

| Yoghurt jelly containing yeast cell wall | |
|---|---|
| Water | 60 g |
| Yeast cell wall (BYC1) | 6 g |

(2) Biscuit containing the yeast cell wall as shown in Table 5 was produced using [BYC1] prepared in Example 2.

Table 5

| Biscuit containing yeast cell wall | |
|---|---|
| Weak flour | 90.0 g |
| Corn starch | 28.0 g |
| Whole egg | 45.0 g |
| Sucrose | 42.0 g |
| Butter | 66.0 g |
| Baking powder | 0.6 g |
| Water | 17.4 g |
| Yeast cell wall (BYC1) | 5.0 g |

Industrial Applicability

The composition of the present invention has skin condition-improving effects, including skin-moistening effects, skin-whitening effects, and active oxygen-elimination effects. Thus, the composition of the present invention can be used for a pharmaceutical product, a food or beverage product, or a feed for improving skin conditions.

All publications, patents, and patent applications cited herein are incorporated herein by reference in their entirety.

**Claims**

1.  A composition for improvement of skin conditions comprising, as an active ingredient, a yeast cell wall fraction obtained by removing soluble intracellular components from an enzymatically treated and/or non-enzymatically treated yeast.

2.  The composition according to claim 1, wherein the active ingredient is a yeast cell wall fraction obtained by further subjecting the yeast cell wall fraction obtained by removing soluble intracellular components from an enzymatically treated and/or non-enzymatically treated yeast, to water washing, or to both high-pressure treatment and water washing.

3.  The composition according to claim 1, wherein the dietary fiber content of the yeast cell wall fraction is 60% by dry weight or more.

4.  The composition according to claim 1, which is fed or administered to an animal.

5.  The composition according to claim 1, wherein the improvement of skin conditions is a skin-moistening effect.

6.  The composition according to claim 1, wherein the improvement of skin conditions is a skin-whitening effect.

7.  The composition according to claim 1, wherein the improvement of skin conditions is an active oxygen-removing effect.

8.  The composition according to claim 1, which is a pharmaceutical product, food or beverage product, pet food, or feed.

9. A method for improving skin conditions of an animal comprising feeding or administering the composition according to claim 1 to an animal.

10. Use of a yeast cell wall fraction obtained by removing soluble intracellular components from an enzymatically treated and/or non-enzymatically treated yeast, for production of a composition for improving skin conditions.

# Fig. 1

# Fig. 2

# Fig. 3

# Fig. 4

# Fig. 5

# Fig. 6

# Fig. 7

# Fig. 8

# Fig. 9

# Fig. 10

## INTERNATIONAL SEARCH REPORT

| | International application No. |
|---|---|
| | PCT/JP2006/316654 |

**A. CLASSIFICATION OF SUBJECT MATTER**
*A61K36/06*(2006.01)i, *A23K1/16*(2006.01)i, *A23L1/30*(2006.01)i, *A61P17/00*(2006.01)i, *A61P17/16*(2006.01)i

According to International Patent Classification (IPC) or to both national classification and IPC

**B. FIELDS SEARCHED**

Minimum documentation searched (classification system followed by classification symbols)
A61K36/06, A23K1/16, A23L1/30, A61P17/00, A61P17/16

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)
WPI, JMEDPlus(JDream2), JST7580(JDream2), JSTPlus(JDream2)

**C. DOCUMENTS CONSIDERED TO BE RELEVANT**

| Category* | Citation of document, with indication, where appropriate, of the relevant passages | Relevant to claim No. |
|---|---|---|
| X | JP 2001-55338 A  (Kirin Brewery Co., Ltd.), 27 February, 2001 (27.02.01), Full text; Claim 9; Par. No. [0002]; test examples 4, 5 & US 2002/155126 A1    & US 2003/118607 A1 | 1-8,10 |
| X | JP 2003-277273 A  (Asahi Breweries, Ltd.), 02 October, 2003 (02.10.03), Full text; Claims; page 2, column 1, lines 33 to 34 (Family: none) | 1-8,10 |
| X | JP 2006-187258 A  (Nisshin Faruma Kabushiki Kaisha), 20 July, 2006 (20.07.06), Full text; Claims; Par. Nos. [0002] to [0003] (Family: none) | 1-8,10 |

| ☒ Further documents are listed in the continuation of Box C. | ☐ See patent family annex. |
|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search 19 April, 2007 (19.04.07) | Date of mailing of the international search report 01 May, 2007 (01.05.07) |
|---|---|
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/316654

C (Continuation). DOCUMENTS CONSIDERED TO BE RELEVANT

| Category* | Citation of document, with indication, where appropriate, of the relevant passages | Relevant to claim No. |
|---|---|---|
| X | JP 2006-502167 A (Biopolymer Engineering, Inc.), 19 January, 2006 (19.01.06), Full text; Claim 7; Par. Nos. [0003] to [0004]; page 8, lines 1 to 2 & WO 04/21994 A2 & AU 2003/268486 A1 & EP 1536820 A2 & US 2006/165700 A1 | 1-8,10 |
| X | Tomohiko MANAMURA, 'Atarashii Beer Kobo Sozai – Beer Kobo Saiboheki [BYC] no Shokuhin Sozai to Shiteno Hoken Kino –', NEW FOOD INDUSTRY, (2003) 45(5), pages 1 to 9, full text; page 5, 3rd line from the bottom to page 7, line 8 | 1-8,10 |
| X | Tomohiko NAKAMURA, 'Beer Kobo Saiboheki [BYC] no Shokuhin Sen'i Sozai to Shiteno Tokusei to Kono', NEW FOOD INDUSTRY, (2001) 43(12), pages 1 to 6, full text; page 5, middle part | 1-8,10 |
| X | MUFTI, J. ET AL. 'IMMUNO-COSMETICS' HOUSEHOLD AND PERSONAL PRODUCTS INDUSTRY, (1996) 33(8) P.41,42,44,46, full text; page 44, right column, lines 11 to 46 | 1-8,10 |
| X | JP 2005-82517 A (Asahi Denka Kogyo Kabushiki Kaisha), 31 March, 2005 (31.03.05), Full text; Claim 3; Par. Nos. [0021] to [0022], [0039], [0050], [0050], [0059] (Family: none) | 1-8,10 |
| X | JP 2005-120100 A (Kirin Brewery Co., Ltd.), 12 May, 2005 (12.05.05), Full text; Claims; page 2, lines 47 to 49; test examples 4, 5 (Family: none) | 1-8,10 |
| X | JP 2004-161618 A (Kirin Brewery Co., Ltd.), 10 June, 2004 (10.06.04), Full text; Claims; page 4, lines 9 to 11 (Family: none) | 1-8,10 |
| X | JP 2005-325065 A (Kirin Brewery Co., Ltd.), 24 November, 2005 (24.11.05), Full text; Claims; Par. No. [0011]; examples 1 to 3 (Family: none) | 1-8,10 |

Form PCT/ISA/210 (continuation of second sheet) (April 2005)

**INTERNATIONAL SEARCH REPORT**

| International application No. |
|---|
| PCT/JP2006/316654 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 6-504191 A  (CPC International Inc.),<br>19 May, 1994 (19.05.94),<br>Full text; Claims; page 3, upper right column,<br>4th line from the bottom to lower left column,<br>line 21; examples 1 to 4, 5, 7<br>& WO 92/07064 A1       & EP 553176 A1<br>& US 5968811 A | 1-8,10 |

Form PCT/ISA/210 (continuation of second sheet) (April 2005)

## EP 2 062 588 A1

**INTERNATIONAL SEARCH REPORT**

International application No.

PCT/JP2006/316654

| Box No. II | Observations where certain claims were found unsearchable (Continuation of item 2 of first sheet) |

This international search report has not been established in respect of certain claims under Article 17(2)(a) for the following reasons:

1. ☒ Claims Nos.: 9
   because they relate to subject matter not required to be searched by this Authority, namely:
   Claim 9 involves embodiments concerning methods for treatment of the human body by therapy and thus relates to a subject matter which this International Searching Authority is not required, under the provisions of Article 17(2)(a)(i) of the PCT      (continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

29

**INTERNATIONAL SEARCH REPORT**

International application No.

PCT/JP2006/316654

Continuation of Box No.II-1 of continuation of first sheet(2)

and Rule 39.1(iv) of the Regulations under the PCT, to search.

Form PCT/ISA/210 (extra sheet) (April 2005)

## REFERENCES CITED IN THE DESCRIPTION

### Patent documents cited in the description

- JP H2134309 A
- JP H995420 A
- JP 2005015348 A
- JP 2005162663 A
- JP 2005185187 A
- JP 2001163720 A
- JP H7278012 A
- JP H11113530 A
- JP 2002262828 A
- JP 2001055338 A

- JP 2002153263 A
- JP 3502341 B
- JP 2004161618 A
- JP 2004292382 A
- JP H856611 A
- JP 1996 A
- JP H956361 A
- JP 1997 A
- JP H11332511 A
- JP 1999 A

### Non-patent literature cited in the description

- Shokuhin bunseki hou. Japanese Society for Food Science and Technology, 101